# EUROPEAN PATENT SPECIFICATION

(11) **EP 1 813 220 B1**
(45) Date of publication and mention of the grant of the patent: **20.10.2010**
(21) Application number: 07001216.6
(22) Date of filing: 19.01.2007
(51) Int. Cl.: A61B 19/02

(54) **Medical device package**
Verpackung für medizinische Vorrichtungen
Emballage de dispositif médical

(30) Priority: 26.01.2006 US 340912
(43) Date of publication of application: 01.08.2007
(60) Divisional application: 10007883.1
(73) Proprietor: Tyco Healthcare Group LP, Norwalk, CT 06856 (US)
(72) Inventor: Stopek, Joshua, Yalesvill CT 06492 (US); Hotter, Joseph, Middletown CT 06457 (US); Cohen, Matthew D., Berlin CT 06037 (US)
(74) Representative: Soames, Candida Jane

(56) References cited:
- EP-A1- 1 316 291
- WO-A-99/37233
- WO-A-03/008285
- WO-A-03/101334

## Description

### BACKGROUND

### Technical Field

The present disclosure relates generally to packaging for medical devices, and more particularly, to a package for a medical device contained within a sealable pouch and a port for delivering at least one agent to the medical device.

### Background of Related Art

Combination medical devices, i.e., medical devices coated with drugs or other bioactive agents, have become more prevalent commercially in recent years. There are many of these combination medical devices known to those skilled in the art. Many of these devices require specialized coatings to facilitate both bioactive agent elution and, more importantly, maintain or enhance the core functionality of the medical device. For example, a suture containing an antimicrobial coating must be able to facilitate the elution of the antimicrobial agent in the coating and also maintain a certain tensile strength, handling ability, knot-tying ability, and degradation rate to ensure the coated suture remains functional as a wound closure device.

Further, with the selection of a new coating, drug or any combination of medical devices comes the challenge of marrying the selected agents with a coating or medical device that can accommodate both technical requirements described above, as well as the manufacturing, sterilizing, and transporting processes involved in producing such products. This often requires the design of new coating polymers, which are specialized to be compatible with a specific agent, as well as new coating, manufacturing, sterilizing and transporting processes. In addition, designing these new coatings and processes creates the added pressures of possibly impacting the shelf-life of the device as well as the end-use of the combination medical device in a negative manner.

Also, medical professionals are limited to using the combination medical device in the dosage and strength produced, without flexibility to alter the product as needed for their respective patients.

WO 03/101334 discloses a device for moistening non-biological medical implant material upon which the two part form of claim 1 is based. WO 03/008285 discloses a device for hydrating and rehydrating orthopedic graft materials. WO 99/37233 discloses an apparatus for aseptically packaging a line material.

Therefore, the present disclosure describes a package for a medical device aimed at simplifying the design and application of combination medical device coatings to provide the following benefits: the ability to choose any bioactive or non-bioactive agent necessary for the individual patient without having to change existing products or manufacturing process; sensitive agents can be delivered without compromising standard shelf or transport conditions; the ability to later combine a specific medical device with agents that were unable to tolerate the required sterilization process for that specific device, under sterile conditions; the medical professional has greater control over product selection; and longer shelf-life of products due to more stable format.

### SUMMARY

Accordingly, a package for a medical device in accordance with the present disclosure includes a sealable pouch and a port positioned adjacent a periphery of the sealable pouch, for permitting the passage of at least one agent to the medical device contained therein. The package also includes a stop member as defined in claim 1.

In another embodiment, the package for a medical device in accordance with the present disclosure may also include an outer, breathable pouch, a sealable pouch and a port positioned adjacent a periphery of the sealable pouch for permitting the passage of at least one agent to the medical device contained therein.

### BRIEF DESCRIPTION OF THE DRAWNGS

Various embodiments are described herein with reference to the drawings wherein:
FIGS. 1A and 1B are a top and end view respectively of a sealable pouch for a medical device;
FIGS. 1C and 1D are a top and side view respectively of a sealable pouch for a medical device;
FIGS. 1E and 1F are a top and end view respectively of a sealable pouch for a medical device;
FIG. 2A is an enlarged top view of a port for permitting the passage of at least one agent to a medical device within a sealable pouch;
FIG. 2B is an enlarged side view of a shunt-like port for permitting the passage of an agent to a medical device within a sealable pouch;
FIG. 2C is an enlarged side view of a shunt-like port as described herein incorporated with a frangible container housing an agent;
FIG. 3 is a top view of a sealable pouch for a medical device containing an inner retainer for securing the medical device which contains a port for permitting the passage of an agent to the medical device within the sealable pouch;
FIG. 4A is a top view of a sealable pouch for a medical device containing a bottom piece of inner retainer including at least one opening and a port positioned within and through sealable pouch 30 that penetrates inner retainer 50 via the at least one opening;
FIG. 4B is a top view of a sealable pouch for a medical device containing a top piece connected to a bottom piece to form the inner retainer including at least one opening and a port positioned within and through sealable pouch 30 that penetrates inner retainer 50 via the at least one opening;
FIG. 5A and 5B are a top and side view of a port positioned within and through an outer breathable pouch, and additionally through a sealable pouch for a medical device, for permitting the passage of an agent to the medical device from the outside of the outer breathable pouch; and
FIG. 6 is a cross-sectional view along line Z₁-Z₂ of FIG. 1E, showing a sealable pouch for a medical device having a port positioned adjacent a periphery of the sealable pouch and a stop member.

### DETAILED DESCRIPTION

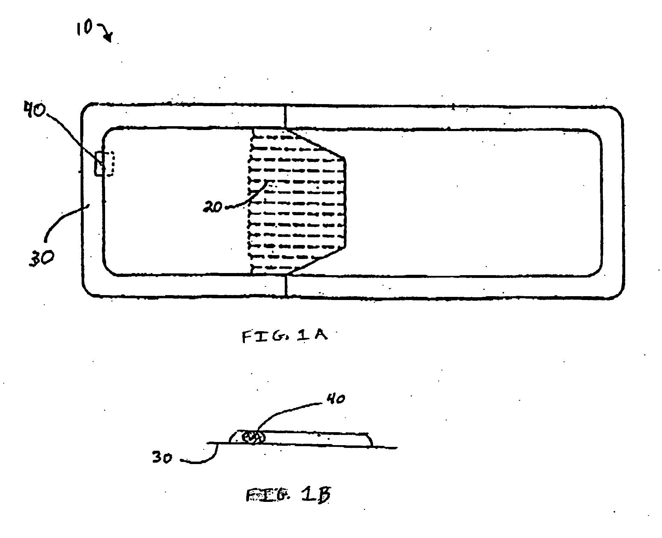

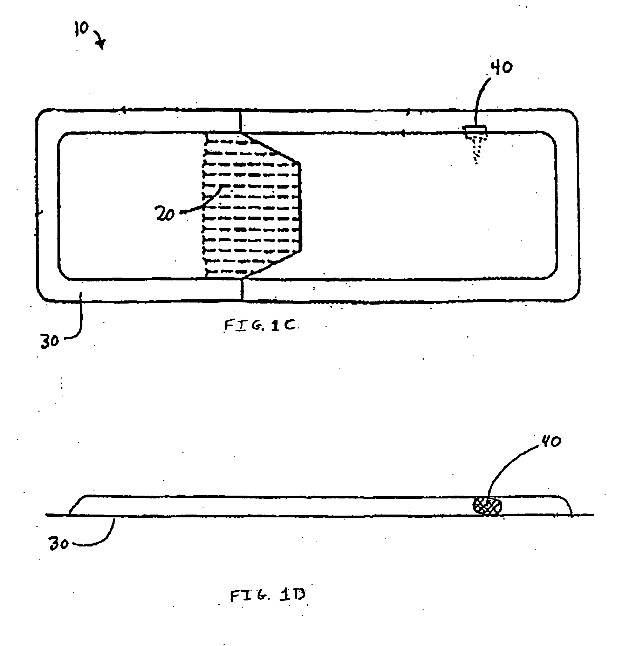

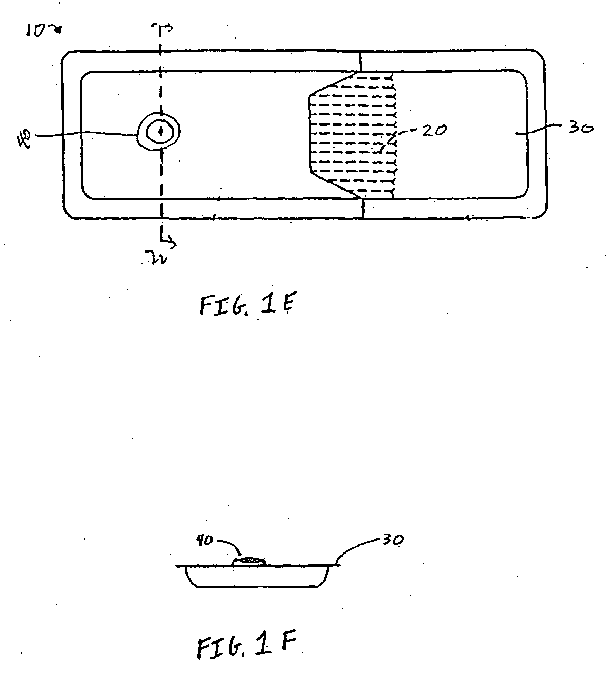

Referring now to FIGS. 1A-1F, package 10 as described herein includes sealable pouch 30 for a medical device 20 and port 40 which is positioned adjacent a periphery of sealable pouch 30 for permitting the passage of at least one agent to the medical device 20 contained therein from outside sealable pouch 30. Any medical device may be stored within package 10. Some examples include, but are not limited to, sutures, staples, clips, adhesives, sealants, meshes, sternum closures, pins, screws, tacks, and adhesion barriers.

The at least one agent may be selected from any bioactive and/or non-bioactive agent suitable for combination with the medical device. Suitable agents include, but are not limited to, drugs, such as antiseptics, anesthetics, muscle relaxants, antihistamines, decongestants, antimicrobial agents, anti-viral agents, anti-fungal agents, antimalarials, amebicides, antituberculosal agents, antiretroviral agents, leprostatics, antiprotazoals, antihelmitics, antibacterial agents, steroids, hematopoietic agents, antiplatelet agents, anticoagulants, coagulants, thrombolytic agents, hemorrheologic agents, hemostatics, plasma expanders, hormones, sex hormones, uterine-active agents, bisphosphonates, antidiabetic agents, glucose-elevating agents, growth hormones, thyroid hormones, inotropic agents, antiarrhythmic agents, calcium channel blockers, vasodilators, sympatholytics, antihyperlipidemic agents, vasopressors, angiotensin antagonists, sclerosing agents, anti-impotence agents, urinary alkanizers, urinary acidifiers, anticholinergics, diuretics, bronchodilators, surfactants, antidepressants, antipsychotics, antianxiety agents, sedatives, hypnotics, barbiturates, antiemetic agents, analgesics, stimulants, anticonvulsants, antiparkinson agents, proton pump inhibitors, H₂-antagonists, antispasmodics, laxatives, antidiarrheals, antiflatulents, digestive enzymes, gallstone solubilizing agents, antihypertensive agents, cholesterol-lowering agents, radiopaque agents, immune globulins, monoclonal antibodies, antibodies, antitoxins, antivenins, immunologic agents, anti-inflammatory agents, antineoplastic agents, alkylating agents, antimetabolites, antimitotic agents, radiopharmaceuticals, vitamins, herbs, trace elements, amino acids, enzymes, chelating agents, immunomodulatory agents and immunosuppressive agents; coating materials such as lubricants, and non-bioabsorbable substances such as silicone, beeswax, or polytetrafluoroethylene, as well as absorbable substances such as collagen, chitosan, chitin, carboxymethylcellulose, and homopolymers and/or copolymers of polyalkylene glycols, and higher fatty acids or salts or esters thereof, glycolic acid, a glycolide, lactic acid, a lactide, p-dioxanone, valerolactone and other lactones derived from linear aliphatic hydroxycarboxylic acids, α-hydroxybutyric acid, ethylene carbonate, ethylene oxide, propylene oxide, propylene carbonate, malic acid ester lactones, succinic acid, adipic acid and other linear aliphatic dicarboxylic acids, and linear aliphatic diols such as butanediol and hexanediol; diluents, such as sterile saline, sterile dextrose in water and sterile lactated ringers; wound healing agents; adhesives; sealants; blood products; blood components; preservatives; colorants; dyes; ultraviolet absorbers; ultraviolet stabilizers; photochromic agents; anti-adhesives; proteins; polysaccharides; peptides; genetic material; viral vectors; nucleic acids; nucleotides; plasmids; lymphokines; radioactive agents; metals; alloys; salts; growth factors; growth factor antagonists; cells; hydrophobic agents; hydrophilic agents; immunological agents; anti-colonization agents; diagnostic agents; imaging agents; and combinations thereof.

Sealable pouch 30 can be any conventional envelope for medical devices manufactured from any suitable material known to those skilled in the art. In one embodiment, sealable pouch 30 is formed by heat sealing two panels of aluminum foil coated on the interior surfaces thereof with a heat sealable polymeric composition. The envelope is bonded around the periphery of the inner sealable pouch as illustrated in FIGS. 1A-1F and 3-6. Other means for sealing the pouch may be employed as is well known to those skilled in the art.

In another embodiment, sealable pouch 30 may be formed from a hydrophobic material. The term "hydrophobic", as described herein, refers to materials that are not normally water soluble and absorb relatively low amounts of water, i.e., less than about 10% by weight. Some examples of these materials include, but are not limited to, polymers, copolymers, homopolymers, and block copolymers formed from monomers such as ε-caprolactone, glycolide, 1-lactide, d,1-lactide, d-lactide, meso-lactide, trimethylene carbonate, 4,4-dimethyl-1,3-dioxan-2-one, p-dioxanone, dioxepanone, δ-valerolactone, β-butyrolactone, ε-decalactone, 2,5-diketomorpholine, pivalolactone, α,α-diethylpropiolactone, 6,8-dioxabicyclooctan-7-one, ethylene carbonate, ethylene oxalate, 3-methyl-1,4-dioxane-2,5-dione, 3,3-dimethyl-1,4-dioxane-2,5-dione, and other subtituted glycolides, and substituted lactides. Some additionally useful hydrophobic materials include polyolefins and polysiloxanes.

It is envisioned that the hydrophobic material can be used to repel the agent that is aqueous away from the sealable pouch thereby forcing the aqueous agent into and around the medical device increasing the wetting or coating of the device. It is envisioned that the sealable pouch's ability to repel an aqueous agent will increase as the material used to form the pouch becomes more hydrophobic.

In another embodiment, sealable pouch 30 may be formed from a hydrophilic material. The term "hydrophilic", as described herein, refers to materials that are normally water soluble and absorb relatively high amounts of water. Some examples of these materials include, but are not limited to, polyalkylene glycols, such as polyethylene glycol, polyacrylates such as polymers of methacrylates and 2-hydroxyethyl methylacrylate, aminoalkyl acrylates, such as N,N-dimethylacrylamide, polyvinylalcohols, polyvinylpyrrolidones, polyoxyethylenes, polyacrylamides, poly(2-hydroxy-ethylmethacrylate), polymethacrylamide, dextran, alginic acid, sodium alginate, polysaccharides, gelatine and copolymers of two or more of the monomers from which the above polymers are derived and polyoxyethylene/polyoxypropylene block copolymers.

It is envisioned that the hydrophilic material can be used to assist in wicking the agent into the package and/or the medical device. It is envisioned that the sealable pouch's ability to wick an agent into the package and medical device will increase as the material used to form the pouch becomes more hydrophilic.

In still another embodiment, sealable pouch 30 can be made of a combination of hydrophobic and hydrophilic materials. It is envisioned that a combination of materials will allow certain areas of the sealable pouch to repel the agent while other areas of the pouch wick the agent towards the medical device. This combination of hydrophobic and hydrophilic materials may be useful when it is desired that certain areas of the pouch and/or medical device receive more of the agent than other areas of the pouch and/or medical device.

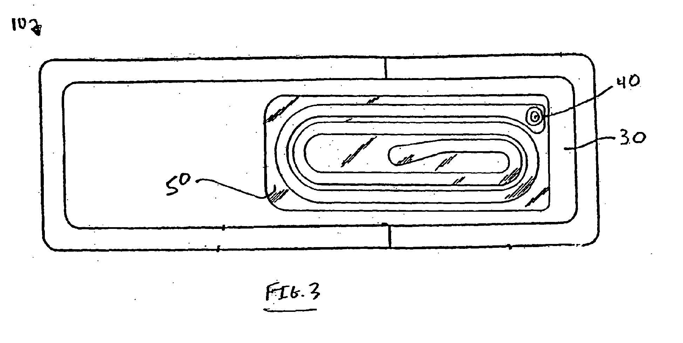

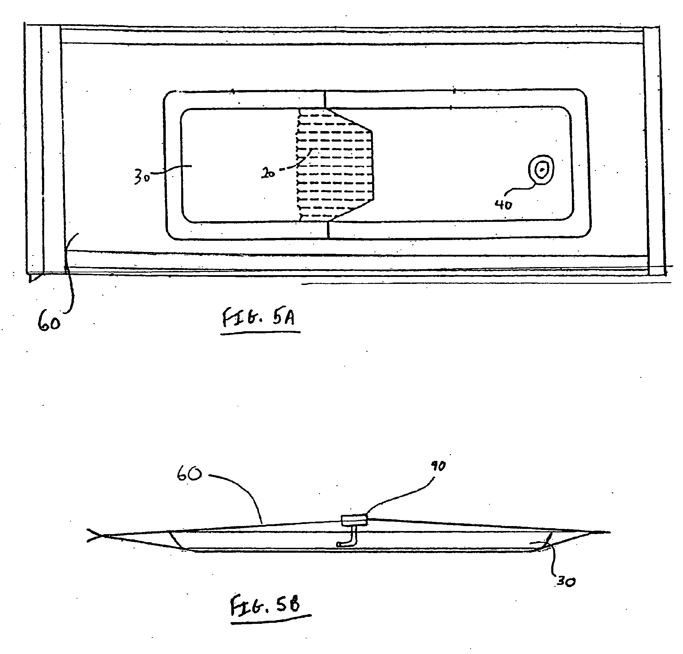

In addition to sealable pouch 30, package 10 includes a sealable port 40. Port 40 is positioned adjacent a periphery of sealable pouch 30, and is designed for delivering at least one agent to medical device 20 from outside sealable pouch 30. As shown in FIGS. 1A-1F, port 40 may be positioned along any side, edge or corner of sealable pouch 30. In addition, port 40 may be considered adjacent a periphery of sealable pouch 30 in a variety of other positions. In one such non-limiting example, port 40 may be positioned anywhere along the outer or inner surface of inner retainer 50 as shown in FIG. 3, wherein port 40 is adjacent the inner periphery of sealable pouch 30. In another non-limiting example, port 40 may be positioned on the outer or inner surface of outer, breathable package 60, as shown in FIGS. 5A-5B, wherein port 40 is adjacent the outer periphery of sealable pouch 30.

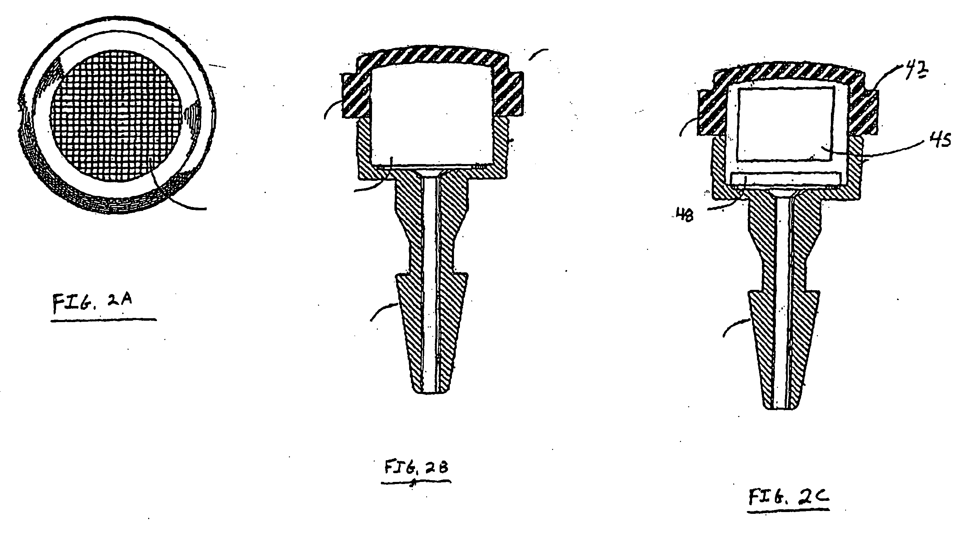

Now turning to FIG. 2A, port 40 is shown as an injectable-hub which is designed to remain sealed by self-sealing action to ensure no fluid medium can escape and also so no pathogens can breach the sealable pouch. Port 40 can be composed of a traditional rubber or thermoplastic material known to be used in sealing sterile vials, intravenous bags, catheters, drug ampules or blood bags. Alternatively, port 40 may be composed of hydrophobic, hydrophilic or a combination of hydrophobic and hydrophilic materials. Suitable ports can be made of any size, shape or dimension.

In one embodiment, port 40 may be a hub designed in such a way that only a particular syringe can mate with port 40 thereby creating a lock and key type of hub to promote only specific use of port 40. This type of port provides more safety to the user of port 40 because the port does not necessarily require the use of a needle. In addition, the lock and key type of hub may be used by patients and medical staff for only certain medications and dosages of those medications, thereby reducing the likelihood of administering the wrong agent or the wrong dosage of the intended agent.

As shown in FIGS. 1C, 1D, 2B, 2C, 4A, 4B, 5A and 5B, port 40 may alternatively be a shunt, whereby port 40 is not only positioned adjacent the periphery of sealable pouch 30, but port 40 is also capable of penetrating into sealable pouch 30. By penetrating into sealable pouch 30, port 40 can direct the injected agent into any specific area within sealable pouch 30 including particular areas of the medical device 20 or inner retainer 50. In addition, the shunt can be positioned to further penetrate the medical device 20 or inner retainer 50 to increase the wetting rate of medical device 20 and/or limit the agent to only certain areas of the medical device 20 or inner retainer 50.

In another embodiment, and as shown in FIG. 2C, port 40 may be incorporated with a frangible container 45 which can be filled with at least one agent either prior to or following the production of the package and container. In this embodiment, the surgeon or other medical staff may apply pressure to the flexible member 42 located on the top of frangible container 45 thereby forcing container 45 to open and release the agent into port 40 which will permit the passage of the bioactive agent into sealable pouch 30. Frangible container 45 may be manufactured from any material known to those skilled in the art. One known example includes glass materials. In embodiments where frangible container 45 is made from glass or other materials not meant to be introduced to the patient, a filter 48 may be connected to port 40, between frangible container 45 and port 40, to keep these materials from entering port 40 following the opening of frangible container 45.

It is envisioned that port 40 may be located anywhere adjacent a periphery of sealable pouch 30, including for example, the inner or outer surfaces of sealable pouch 30. In one embodiment, port 40 may be positioned within and through the surface of sealable pouch 30, inner retainer 50, or outer breathable package 60. In this embodiment, port 40 is sealed within the surface of sealable pouch 30, inner retainer 50 or outer breathable retainer 60 and passes through into the selected retainer or pouch. In another embodiment, port 40 may be positioned only on the surface of sealable pouch 30, inner retainer 50, or outer breathable package 60. It is envisioned that wherein port 40 is positioned only on the surface of one of the retainers, port 40 may be applied to the surface using any medical adhesive suitable for attaching port 40 to the surface. It is further envisioned that port 40 may be adhered to a surface with an adhesive that allows port 40 to be easily peeled away from the surface and adhered to another surface of the pouch, thereby allowing port 40 to be repositioned to another surface within package 10.

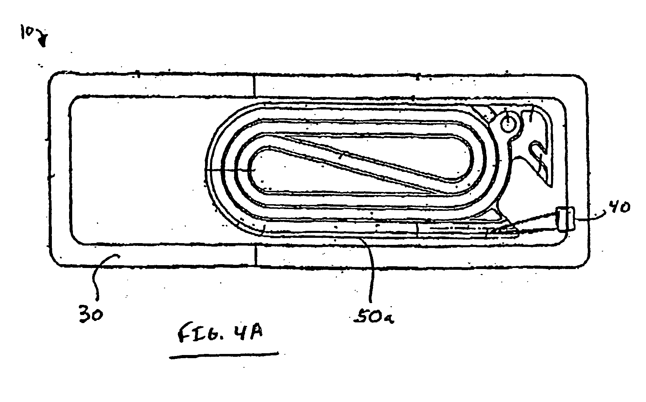

In addition to sealable pouch 30 and port 40, package 10 as described herein may further contain an inner retainer 50. Inner retainer 50 is designed to store the medical device in a certain position depending upon the individual medical device being stored. For example, inner retainer 50 may contain channels, as shown in FIGS. 3 and 4A (commonly known as a race track-type retainer) wherein a medical device such as a suture can be placed thereby preventing the suture from becoming entangled.

Inner retainer 50 can be made of one or more pieces of any sterilizable material suitable for positioning a medical device. Some examples include hydrophobic materials, hydrophilic materials, paper materials or a combination of these materials. One example of paper materials includes a heavy weight, relatively stiff paper or paperboard such as 5 point to 12 point solid, bleached sulfate board. This paperboard is readily foldable and yet sufficiently strong and stiff to support the medical device and provide a relatively rigid package. Additional materials include plastics, foils and laminates.

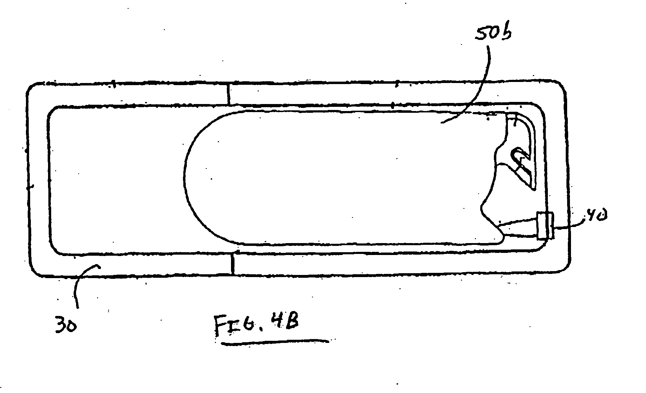

Now turning to FIGS. 4A and 4B, inner retainer 50 is shown including a top piece 50a and a bottom piece 50b, and at least one opening to allow port 40 or the at least one agent to penetrate into retainer 50 and coat medical device 20. Inner retainer 50 can be sealable or non-sealable.

Turning now to FIGS. 5A and 5B, package 10 may also further include outer breathable pouch 60 which is designed to protect sealable pouch 30 during the sterilizing and transporting processes. Outer breathable pouch 60 may be made from breathable materials known to those skilled in the art. Suitable materials for outer breathable pouch 60 exhibit the combined effects of good permeability to sterilize gases and adequate barrier efficacy in order to prevent the entry of bacteria into package 10. Generally, cellulosic paper-based webs are sufficently breathable to allow for gas sterilization techniques but yet sufficiently impervious to prevent certain bacteria, spores and other microorganisms from passing through. Some examples include, but are not limited to, cellulosic fibers, polyethylene, polypropylene, Tyvek®, and combinations thereof.

As shown in FIGS. 5A and 5B, package 10 includes a port 40 positioned within and through outer breathable pouch 60 and passing into sealable pouch 30 which is sealed within outer breathable pouch 60 and may contain inner retainer 50 and/or medical device 20. In these embodiments, the at least one agent may be permitted to pass from outside outer, breathable pouch 60 into sealable pouch 30 without breaching outer, breathable pouch 60 or sealable pouch 30.

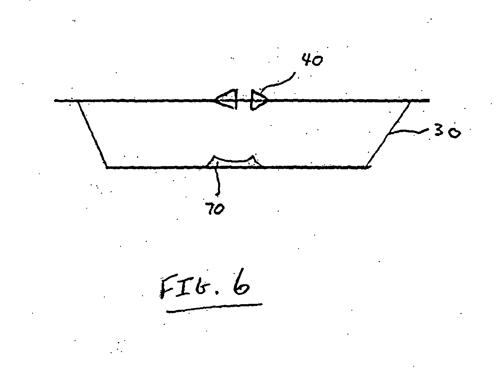

In addition to port 40, sealable pouch 30 further contains a stop-member 70, as shown in FIG. 6, which is a cross-section of pouch 30 along dotted line Z1-Z2 in FIG. 1E. Stop-member 70 is designed to prevent a needle or other relatively sharp delivery device from passing through port 40 and exiting out the surface directly opposite port 40 on the sealable pouch 30, inner retainer 50 or outer breathable pouch 60. Stop-member may be made of any material hard enough to prevent the sharpened delivery device from passing through the opposite surface. Some examples include, but are not limited to, polymeric materials and metal alloys. Similar to port 40, stop-member 70 may be positioned on or within and through any side, edge, corner or surface of package 10. Stop-member 70 is positioned in direct alignment with port 40 and on a surface located behind the expected point of entry of port 40. In addition, stop-member 70 may be formed into any shape conducive to preventing the relatively sharp delivery device from passing through, including a flat-block design or a concave or bowl design as shown in FIG. 6.

In other embodiments, sealable pouch 30 may contain medical device 20 and an agent in a particulate or powdered format. In this dry format, the agent remains stable inside sealable pouch 30 for longer periods of time thereby improving the products shelf-life and can be reconstituted at any time prior to opening the sealed pouch. The agent in powder form can be reconstituted by injecting a sterile diluent into sealable pouch 30 via port 40 prior to use of the packaged medical device 20. As stated above, some known diluents include sterile saline solution, sterile dextrose in water, and sterile lactated ringers. It is also envisioned that any other agent may be added to the dry powder form inside the sealable pouch to form a suspension, solution, dispersion, gel, film, sheet, etc. as needed to enhance the medical device sealed therein.

It will be evident from the above description what is meant by "port". Drawing Figures 2A, 2B and 2C reveal constructions that one can recognise as having a septum through which a hollow needle with a pointed tip can be advanced, for injecting a liquid into a space below the septum via the bore of the needle, with the septum sealing itself, at the point of needle puncture, upon withdrawal of the needle. The port will in general display a rim encircling a port area (usually circular), the rim being relatively inflexible, with any septum sealing the area encircled by the rim. That area will generally correspond to a cavity, and extending away from this cavity and from the area and from any septum may be a flow channel in a flow conduit that can be in the nature of a spigot, extending from the port as such. Such spigot might have an abluminal surface that displays detent portions that engage with the pouch to permit the spigot to be advanced into the pouch but to resist reverse movement that might otherwise occur upon attempting to pull the port out of the pouch.

## Claims

1. A medical device package comprising:
a sealable pouch (30);
a sealed port (40) in the sealable pouch, which enables the passage of an agent from outside the sealable pouch (30) to a medical device (20) sealed within the pouch (30); **characterised by**
a stop member (70) for preventing a relatively sharp delivery device advanced through the port (40) from adversely affecting the surface of the pouch lying opposite the port (40).

2. The package of claim 1, wherein the port (40) includes a puncturable septum.

3. The package of claim 1 or 2, wherein the port (40) is a shunt.

4. The package of any one of the preceding claims, wherein the sealable pouch (30) comprises aluminium foil.

5. The package of any one of the preceding claims, wherein the sealable pouch (30) incorporates a hydrophobic material.

6. The package of any one of the preceding claims, wherein the sealable pouch (30) incorporates a hydrophilic material.

7. Package as claimed in any one of the preceding claims, wherein the stop member (70) is located on the said surface opposite the port (40).

8. Package as claimed in any one of the preceding claims, wherein the port (40) is on the periphery of the pouch (30).

9. Package as claimed in any one of claims 1 to 7, wherein the port (40) is near the periphery of the pouch (30).

10. Package as claimed in any one of the preceding claims, that contains a medical device (20) and wherein that medical device (20) is laterally spaced away from the port (40).

11. Package as claimed in claim 10, in which the medical device (20) extends around the periphery of the cavity defined by the pouch, and the port (40) is located near the centre of the cavity so that the device does not lie below the port (40).

12. The package of any one of the preceding claims, and further comprising an inner retainer (50) for positioning the medical device (20) within the sealable pouch (30).

13. The package of claim 12, wherein the port (40) is a positioned within and through the inner retainer (50).

14. The package of claim 12 or 13, wherein the port (40) is positioned on the surface of the inner retainer (50).

15. The package of claim 12, 13 or 14, wherein the port (40) is a shunt that penetrates the inner retainer (50).

16. The package of any one of the preceding claims, and further comprising a frangible container (45) suitable to contain the agent, wherein the frangible container (45) is incorporated with the sealed port (40) whereby the agent can pass from the frangible container (45) to the medical device (20) contained inside the sealable pouch (30) upon the opening of the frangible container (45).

17. The package of claim 16, wherein the frangible container (45) is a breakable ampule.

18. The package of claim 16 or 17, and further comprising a filter (48) to keep material making up the frangible container (45) from passing to the medical device (20) following opening of the frangible container (45).

19. The package of any one of the preceding claims, and further comprising an outer, breathable pouch (60) for storing the sealable pouch (30) and the medical device (20).

20. The package of claim 20, wherein the port (40) is a positioned within and through the outer, breathable pouch (60).

21. The package of claim 19 or 20, wherein the port (40) is positioned on the surface of the outer, breathable pouch (60).

22. The package of any one of claims 19 to 21, wherein the port (40) is positioned within and through the sealable pouch (30) and the outer breathable pouch (60) for permitting the passage of at least one agent to the medical device (20) from outside the outer breathable pouch (60).

23. A method of packaging a medical device (20) comprising:
providing a medical device (20); and
placing the medical device (20) in the package of claim 1.

24. A method according to claim 23 comprising the steps of:
i) sealing the medical device (20) inside the sealable pouch (30) that is equipped with a port (40), and
ii) contacting the medical device (20) with an agent, by advancing said agent through the port (40) into a cavity within the sealed pouch (40) that contains the medical device (20),
wherein the step of advancing is accomplished by advancing a needle shaft through a septum of the port, and then advancing the agent through the port via a bore in the needle shaft, and wherein the needle shaft advanced through the port, by the stop member (70), from adversely affecting the surface of the pouch lying opposite the pouch.

25. Method according to claim 24, wherein the step of advancing is further accomplished by fracturing a frangible container (45) within the port (40) that contains said agent.

26. The package of any one of claims 1 to 22, wherein the port (40) is adhered to a surface of the pouch (30) with an adhesive that allows port (40) to be peeled away from the surface and adhered to another surface of the pouch (30), thereby allowing the port (40) to be repositioned.

27. The package of any one of claims 1 to 22 or 26, wherein the sealable pouch (30) is formed by heat sealing two panels of aluminium foil coated on the inner surfaces thereof with a heat sealable polymeric composition, wherein the pouch (30) in bonded around the periphery of the pouch to seal the pouch.

## Patentansprüche

1. Eine Verpackung für medizinische Vorrichtungen, umfassend:
einen abdichtbaren Beutel (30);
einen abgedichteten Zugang (40) in dem abdichtbaren Beutel, welcher das Hindurchtreten eines Mittels von außerhalb des abdichtbaren Beutels (30) zu einer medizinischen Vorrichtung (20), die in dem Beutel (30) abgedichtet ist, ermöglicht; **gekennzeichnet durch**
ein Stoppelement (70) zum Verhindern, dass eine relativ scharfe bzw. spitze Zuführvorrichtung, die **durch** den Zugang (40) geschoben wurde, die Oberfläche des Beutels, die dem Zugang (40) gegenüberliegend angeordnet ist, nachteilig beeinflusst.

2. Die Verpackung nach Anspruch 1, wobei der Zugang (40) ein durchstechbares Septum umfasst.

3. Die Verpackung nach Anspruch 1 oder 2, wobei der Zugang (40) ein Shunt ist.

4. Die Verpackung nach einem der vorstehenden Ansprüche, wobei der abdichtbare Beutel (30) Aluminiumfolie umfasst.

5. Die Verpackung nach einem der vorstehenden Ansprüche, wobei der abdichtbare Beutel (30) ein hydrophobes Material enthält.

6. Die Verpackung nach einem der vorstehenden Ansprüche, wobei der abdichtbare Beutel (30) ein hydrophiles Material enthält.

7. Verpackung nach einem der vorstehenden Ansprüche, wobei sich das Stoppelement (70) dem Zugang (40) gegenüberliegend auf der Oberfläche befindet.

8. Verpackung nach einem der vorstehenden Ansprüche, wobei der Zugang (40) sich an dem Rand des Beutels (30) befindet.

9. Verpackung nach einem der vorstehenden Ansprüche, wobei sich der Zugang (40) in Nähe des Randes des Beutels (30) befindet.

10. Verpackung nach einem der vorstehenden Ansprüche, die eine medizinische Vorrichtung (20) enthält und wobei die medizinische Vorrichtung (20) lateral von dem Zugang (40) beabstandet ist.

11. Verpackung nach Anspruch 10, wobei sich die medizinische Vorrichtung (20) um den Rand des Hohlraums, der durch den Beutel definiert ist, erstreckt, und wobei sich der Zugang (40) in Nähe des Zentrums des Hohlraumes befindet, sodass die Vorrichtung nicht unterhalb des Zugangs (40) liegt.

12. Die Verpackung nach einem der vorstehenden Ansprüche, des Weiteren eine innere Halteeinrichtung (50) zum Positionieren der medizinischen Vorrichtung (20) innerhalb des abdichtbaren Beutels (30) umfassend.

13. Die Verpackung nach Anspruch 12, wobei der Zugang (40) innerhalb und durch die innere Halteeinrichtung (50) positioniert ist.

14. Die Verpackung nach Anspruch 12 oder 13, wobei der Zugang (40) auf der Oberfläche der inneren Halteeinrichtung (50) positioniert ist.

15. Die Verpackung nach Anspruch 12, 13 oder 14, wobei der Zugang (40) ein Shunt ist, der die innere Halteeinrichtung (50) durchdringt.

16. Die Verpackung nach einem der vorstehenden Ansprüche, des Weiteren einen zerbrechlichen Behälter (45) umfassend, der geeignet ist, das Mittel zu enthalten, wobei der zerbrechliche Behälter (45) in den abgedichteten Zugang (40) integriert ist, wodurch das Mittel von dem zerbrechlichen Behälter (45) zu der medizinischen Vorrichtung (20), die in dem abdichtbaren Beutel (30) enthalten ist, nachfolgend auf das Öffnen des zerbrechlichen Behälters (45) übertreten kann.

17. Die Verpackung nach Anspruch 16, wobei der zerbrechliche Behälter (45) eine zebrechbare Ampulle ist.

18. Die Verpackung nach Anspruch 16 oder 17, des Weiteren einen Filter (48) umfassend, um das Material, das den zerbrechlichen Behälter (45) ausmacht, daran zu hindern, nachfolgend auf ein Öffnen des zerbrechlichen Behälters (45) zu der medizinischen Vorrichtung (20) überzutreten.

19. Die Verpackung nach einem der vorstehenden Ansprüche, des Weiteren einen äußeren, atmungsaktiven Beutel (60) zum Aufnehmen des abdichtbaren Beutels (30) und der medizinischen Vorrichtung (20) umfassend.

20. Die Verpackung nach Anspruch 19, wobei der Zugang (40) innerhalb und durch den äußeren, atmungsaktiven Beutel (60) positioniert ist.

21. Die Verpackung nach Anspruch 19 oder 20, wobei der Zugang (40) auf der Oberfläche des äußeren, atmungsaktiven Beutels (60) positioniert ist.

22. Die Verpackung nach Anspruch 19 bis 21, wobei der Zugang (40) innerhalb und durch den abdichtbaren Beutel (30) und den äußeren, atmungsaktiven Beutel (60) positioniert ist, um das Hindurchtreten von mindestens einem Mittel zu der medizinischen Vorrichtung (20) von außerhalb des äußeren, atmungsaktiven Beutels (60) zu ermöglichen.

23. Ein Verfahren zum Verpacken einer medizinischen Vorrichtung (20), umfassend:
Bereitstellen einer medizinischen Vorrichtung (20); und
Platzieren der medizinischen Vorrichtung (20) in der Verpackung nach Anspruch 1.

24. Ein Verfahren nach Anspruch 23, die folgenden Schritte umfassend:
i) Abdichten der medizinischen Vorrichtung (20) innerhalb des abdichtbaren Beutels (30), der mit einem Zugang (40) ausgestattet ist, und
ii) in Kontakt Bringen der medizinischen Vorrichtung (20) mit einem Mittel, durch Befördern des Mittels durch den Zugang (40) in einen Hohlraum innerhalb des abgedichteten Beutels (40) der die medizinische Vorrichtung (20) enthält,
wobei der Schritt des Beförderns erzielt wird durch Schieben eines Nadelschafts durch ein Septum des Zugangs, und anschließendes Befördern des Mittels durch den Zugang mittels eines Lochs in dem Nadelschaft, und wobei der Nadelschaft, der durch den Zugang geschoben wurde, von dem Stoppelement (70) gehindert wird, die Oberfläche des Beutels, die dem Zugang gegenüberliegend angeordnet ist, nachteilig zu beeinflussen.

25. Verfahren nach Anspruch 24, wobei der Schritt des Beförderns des Weiteren erzielt wird durch Zerbrechen eines zerbrechlichen Behälters (45) innerhalb des Zugangs (40), der das Mittel enthält.

26. Die Verpackung nach einem der Ansprüche 1 bis 22, wobei der Zugang (40) an eine Oberfläche des Beutels (30) mit einem Klebstoff angeheftet ist, der es ermöglicht, den Zugang (40) von der Oberfläche abzulösen und an eine andere Oberfläche des Beutels (30) anzuheften, wodurch ein Repositionieren des Zugangs (40) ermöglicht wird.

27. Die Verpackung nach einem der Ansprüche 1 bis 22 oder 26, wobei der abdichtbare Beutel (30) durch Wärmeversiegelung von zwei Panelen von Aluminiumfolie, die an ihren inneren Oberflächen mit einer wärmeversiegelbaren polymerischen Zusammensetzung beschichtet sind, ausgebildet wird, wobei der Beutel (30) um den Rand des Beutels verbunden wird, um den Beutel abzudichten.

## Revendications

1. Emballage de dispositif médical comprenant :
une poche scellable (30) ;
une ouverture scellée (40) dans la poche scellable, permettant le passage d'un agent depuis l'extérieur de la poche scellable (30) vers un dispositif médical (20) scellé à l'intérieur de la poche (30) ; **caractérisé par**
un élément d'arrêt (70) pour empêcher qu'un dispositif de fourniture relativement tranchant introduit dans l'ouverture (40) n'affecte défavorablement la surface de la poche qui se trouve à l'opposé de l'ouverture (40).

2. Emballage selon la revendication 1, dans lequel l'ouverture (40) comporte un septum perforable.

3. Emballage selon la revendication 1 ou 2, dans lequel l'ouverture (40) est une dérivation.

4. Emballage selon l'une quelconque des revendications précédentes, dans lequel la poche scellable (30) comprend une feuille d'aluminium.

5. Emballage selon l'une quelconque des revendications précédentes, dans lequel la poche scellable (30) contient une matière hydrophobe.

6. Emballage selon l'une quelconque des revendications précédentes, dans lequel la poche scellable (30) contient une matière hydrophile.

7. Emballage selon l'une quelconque des revendications précédentes, dans lequel l'élément d'arrêt (30) est situé sur ladite surface opposée à l'ouverture (40).

8. Emballage selon l'une quelconque des revendications précédentes, dans lequel l'ouverture (40) est sur la périphérie de la poche (30).

9. Emballage selon l'une quelconque des revendications 1 à 7, dans lequel l'ouverture (40) est proche de la périphérie de la poche (30).

10. Emballage selon l'une quelconque des revendications précédentes, contenant un dispositif médical (20) et dans lequel ce dispositif médical (20) est éloigné latéralement de l'ouverture (40).

11. Emballage selon la revendication 10, dans lequel le dispositif médical (20) s'étend autour de la périphérie de la cavité définie par la poche et l'ouverture (40) est située près du centre de la cavité de façon que le dispositif ne se trouve pas au-dessous de l'ouverture (40).

12. Emballage selon l'une quelconque des revendications précédentes et comprenant en outre un dispositif de retenue intérieur (50) pour positionner le dispositif médical (20) à l'intérieur de la poche scellable (30).

13. Emballage selon la revendication 12, dans lequel l'ouverture (40) est positionnée à l'intérieur du dispositif de retenue intérieur (50) et à travers celui-ci.

14. Emballage selon la revendication 12 ou 13, dans lequel l'ouverture (40) est positionnée sur la surface du dispositif de retenue intérieur (50).

15. Emballage selon la revendication 12, 13 ou 14, dans lequel l'ouverture (40) est une dérivation qui pénètre dans le dispositif de retenue intérieur (50).

16. Emballage selon l'une quelconque des revendications précédentes et comprenant en outre un récipient cassable (45) adapté à contenir l'agent, dans lequel le récipient cassable (45) est incorporé avec l'ouverture scellée (40) de façon que l'agent puisse passer du récipient cassable (45) au dispositif médical (20) contenu à l'intérieur de la poche scellable (30) en cas d'ouverture du récipient cassable (45).

17. Emballage selon la revendication 16, dans lequel le récipient cassable (45) est une ampoule brisable.

18. Emballage selon la revendication 16 ou 17 et comprenant en outre un filtre (48) pour empêcher la matière constituant le récipient cassable (45) de passer vers le dispositif médical (20) en cas d'ouverture du récipient cassable (45).

19. Emballage selon l'une quelconque des revendications précédentes et comprenant en outre une poche extérieure perméable à l'air (60) pour contenir la poche scellable (30) et le dispositif médical (20).

20. Emballage selon la revendication 20, dans lequel l'ouverture (40) est positionnée à l'intérieur de la poche extérieure perméable à l'air (60) et à travers celle-ci.

21. Emballage selon la revendication 19 ou 20, dans lequel l'ouverture (40) est positionnée sur la surface de la poche extérieure perméable à l'air (60).

22. Emballage selon l'une quelconque des revendications 19 à 21, dans lequel l'ouverture (40) est positionnée à l'intérieur de la poche scellable (30) et à travers celle-ci et la poche extérieure perméable à l'air (60) pour permettre le passage d'au moins un agent vers le dispositif médical (20) depuis l'extérieur de la poche extérieure perméable à l'air (60).

23. Procédé d'emballage d'un dispositif médical (20) comprenant :
la fourniture d'un dispositif médical (20) ; et
le placement du dispositif médical (20) dans l'emballage selon la revendication 1.

24. Procédé selon la revendication 23, comprenant les étapes consistant à :
i) sceller le dispositif médical (20) à l'intérieur de la poche scellable (30) qui est équipée d'une ouverture (40), et
ii) mettre en contact le dispositif médical (20) avec un agent, en faisant avancer ledit agent à travers l'ouverture (40) dans une cavité située à l'intérieur de la poche scellée (40) contenant le dispositif médical (20),
dans lequel l'étape d'avancement est réalisée en faisant avancer une tige d'aiguille à travers un septum de l'ouverture puis en faisant avancer l'agent à travers l'ouverture par l'intermédiaire d'un alésage dans la tige d'aiguille, et dans lequel la tige d'aiguille avance à travers l'ouverture au moyen de l'élément d'arrêt (70) empêchant d'affecter défavorablement la surface de la poche qui se trouve à l'opposé de la poche.

25. Procédé selon la revendication 24, dans lequel l'étape d'avancement est réalisée en outre en brisant un récipient cassable (45) à l'intérieur de l'ouverture (40) qui contient ledit agent.

26. Emballage selon l'une quelconque des revendications 1 à 22, dans lequel l'ouverture (40) est collée sur une surface de la poche (30) avec un adhésif permettant d'arracher la poche (40) de la surface et de la coller sur une autre surface de la poche (30) de façon à pouvoir repositionner l'ouverture (40).

27. Emballage selon l'une quelconque des revendications 1 à 22 ou 26, dans lequel la poche scellable (30) est réalisé en scellant par chauffage deux panneaux de feuille d'aluminium déposés sur ses surfaces intérieures avec une composition polymère scellable à chaud, dans lequel la poche (30) est liée autour de la périphérie de la poche afin de sceller la poche.
